# EUROPEAN PATENT APPLICATION

(11) **EP 2 006 321 A1**
(43) Date of publication of application: **24.12.2008**
(21) Application number: 08398007.8
(22) Date of filing: 19.06.2008
(51) Int. Cl.: C08H 1/00, A61K 47/00

(54) **Synthesis and application of a family of new materials resulting from the chemical cross-linking between gelatine and organic salts**

(30) Priority: 20.06.2007 PT 10376507
(71) Applicant: Universidade Nova de Lisboa, 1099-085 Lisboa (PT)
(72) Inventor: Barreiros, Susana Filipe, 1070-195 Lisboa (PT); Gomes, Pedro Miguel Vidinha, 2820-539 Charneca da Caparica (PT); Lourenço, Nuno Miguel Torres, 2829-516 Caparica (PT); Cabral, Joaquim Manuel Sampaio, 1800-348 Lisboa (PT); Ribeiro, Inês Isabel Baptista, 2825-085 Monte da Caparica (PT); Afonso, Carlos Alberto Mateus, 1049-001 Lisboa (PT)
(74) Representative: Canelas, Alberto Herminio

(57) **Abstract**

The Scheme (I) illustrates the synthesis mechanism of the new materials. This invention involves the combination of gelatine with organic salts in the solid or liquid state. For that purpose, gelatines of several origins (animal, microbial, vegetal) can be used. 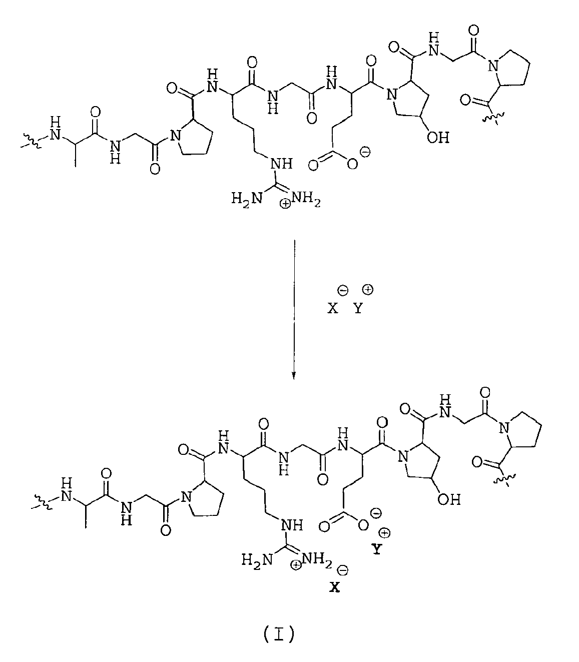 wherein X^{⊖} represents the anion of the salt and Y^{⊕} represents the cation of the salt.

The invention relates also to the application of said synthesized new materials in the domains of physics, chemistry and biology, disclosing the process for the synthesis of the new materials and the use thereof as supports for biocatalysts immobilization in chemical reactions, controlled drug delivery, manufacturing of sensors/biosensors aimed at the detection of chemical species, manufacturing of conductive/semi-conductive materials, electrochemical (galvanic or electrolytic) cells or parts of cells.

## Description

### Field of the invention

This invention relates to the synthesis and application of a family of new materials resulting from the cross-linking between proteins and organic salts. The invention discloses the process for the synthesis of the new materials and the use thereof as supports for biocatalysts immobilization in chemical reactions, controlled drug delivery, manufacturing of sensors/biosensors aimed at the detection of chemical species, manufacturing of conductive/semi-conductive materials, electrochemical (galvanic or electrolytic) cells or parts of cells.

The fields of chemistry and biology, namely the processes for chemical reactions and biological reactions, are within the scope of application of the invention. The industrial sectors targeted for the employment of the new materials include the chemical industry and fine chemistry, bioconversion processes within biotechnology, biological processes in areas such as medicine, controlled drug delivery, cell growth, tissue regeneration, uses as bioadhesive, as electronic devices implant, nanotechnology, uses as biosensor, as conductive/semi-conductive material, as an electrochemical cell or part of cell.

### Background of the Invention

Proteins play a fundamental part in the organization and integrity of biological systems and are responsible for the whole of their organization at the molecular level. The role of proteins is intimately related to their capacity of responding to environmental changes, this being reflected by changes found in the native conformation of proteins, as well as in the aggregation and formation of three-dimensional elastic nets, in a process known as gelification.

From the industrial point of view, gels are extremely important in as much as they can act as coating agents for a different number of compounds. For instance, in the food industry, gels give structure, texture and stability to food products, also providing for the retention of high amounts of water and other small molecules within a food matrix. The most common among these gels is the gelatine one.

Gelatine is a natural polymer that can be prepared from the thermal denaturing of collagen by means of alkaline or acid pre-treatment. Usually, gelatine contains a high number of amino acids in its structure, particularly glycine, proline and 4-hydroxyproline. Gelatine is a heterogeneous mixture of linear and branched polypeptides, each one of them with a triple helical conformation which contains from 300 to 4000 amino acids [1].

Gelatine is commercially available in the free pyrogenic form and is largely used in medical and pharmaceutical applications due to its excellent mechanical and biodegradable properties [2]. These properties make gelatine an extremely safe material for use as an ingredient in pharmaceutical formulations [3], controlled drug delivery, tissue engineering and bioadhesives [4].

In order to enhance some properties, in particular the mechanical and thermal properties of gelatine, a cross-linking is frequently required. Two types of cross-linking can be made: chemical or physical. The chemical one comprises reacting the gelatine with organic molecules such as aldehydes [5], epoxides, isocyanates, acyl azides, carboimides [6], and oxidized sugars [7].

The physical one comprises a heat treatment or irradiation in the UV area, wherein dehydration is carried out. More recent procedures have been described in literature that relate to the controlled drug delivery in tissues, using microwave irradiation techniques which provide a good degree of cross-linking and a favourable response in terms of biodegradability of the material [8].

Another technique that has been largely referred to in the literature for application in the production of nanofibres is the electroplating with a cross-linking by glutaraldehyde vapour, wherein the cross-linking is performed simultaneously to the formation of nanofibres [9] .

These materials can be cross-linked/combined with solvents which are generally known as ionic liquids (IL) because they are fully comprised of ions, the cation commonly being of organic nature and the anion of inorganic or organic nature [10]. Due to the fact that they are composed of ions, these liquids have not a measurable volatility [11]. Their thermal stability (that is above 300°C for many of the known liquids), ionic conductivity, insolubility in supercritical CO₂ (sc CO₂), and low solubility in hexane, ethyl ether and water, allows two-phase systems (biphasic) to be produced and gives them great ease of dissolution as to a wide range of organic and inorganic molecules, and complexes of transition metals (it is particularly known that, depending on the chosen anion and cation, they can solubilise supercritical CO₂, carbonyl compounds, alkyl halides and alcohols). These properties make them an environmentally feasible alternative to the substitution of traditional solvents [10]. Another great advantage of these liquids is the possibility for them to be tailored as appropriate, i.e., their properties can be adjusted according to the reaction requirements.

Ionic liquids can be used in many different applications, namely as recyclable reaction media in chemical processes, including biocatalysis and chemical catalysis [10], biosensors [12] for the capture of CO₂ [12], on the substitution of traditional organic solvents (TOS) in two-phase extractions of the aqueous type - TOS and sc CO₂ TOS [14], as a stationary phase in chromatography [14], for the selective transport using supported liquid membranes [16], in pervaporation [17], on cellulose dissolution [18], as an electrolyte and in fuel cells [19].

In what concerns the most widely used ionic liquids, references are made to several types of cationic unities, in particular, phosphonium [20], ammonium [21] (including chiral [22]), sulfonium [23], piridinium and 8-alkyl-1,8-diazabicyclo[5,4,0]-7-undecene [alkyl-DBU] [24], alkylguanidinium [25], different amino acids [26]; however, the most common unit is, undoubtedly, 1,3-dialkylimidazolium [im] [27]. As far as the anions are concerned, there are references to ClO₄, NO₃, BF₄, PF₆, alkyl [28] and aryl sulphonates, phosphates, carboxylates and even carboranes.

As regards the combination of IL with gelatine, only some documents mention the possibility for both being mixed, although with other compounds and solutes, using chemical processes which are distinct from our approach. The search done in patent databases has shown some references with relevance to this invention. Olson et al. (EP 1315033 Al; EP 1315032 Al) describe the use of IL as coupling solvents in photothermographic systems, and also as image correctors. Watanabe et al. (patent EP 1675211 A1) describe the application of IL for the development of an electrolyte used as photoelectric convertor. This process includes BMIMDCA as one of the IL to be used on these electrolytes.

Baroli et al. (patent US 2006/0222677 Al) disclose a process for the formation of a material to be used as protecting agent of active compounds during a photopolymerization process. In this invention, compounds of gelatine with organic salts are used. However, both the process and the final formulation of the material are clearly distinct from our material. Our invention differs from the aforementioned because in our process for the manufacturing of the material there is no polymerization being employed, since our material results from the cross-linking of the two species.

This invention relates to the synthesis and application of a family of new materials resulting from the cross-linking of both species.

### Detailed description of the invention

The present invention comprises the synthesis and application of a family of new materials resulting from the cross-linking between proteins and organic salts.

The scheme (I) illustrates the synthesis mechanism of said new materials 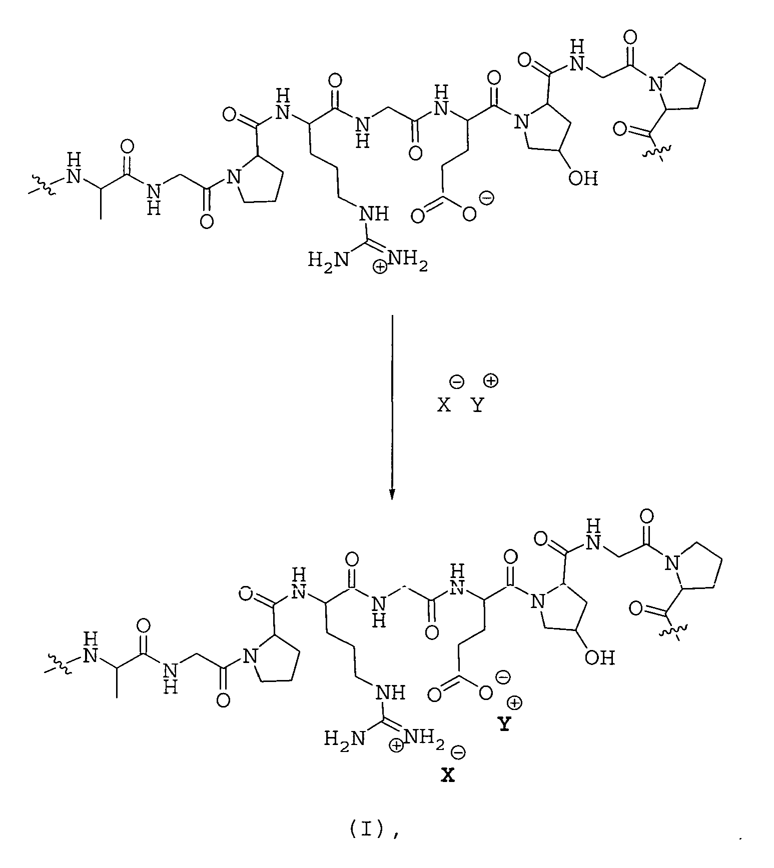 wherein X^{⊖} represents the anion of the salt and Y^{⊕} represents the cation of the salt, and wherein gelatines of several origins (animal, microbial, vegetal) can be used, in association with different types of organic salts. A common procedure for obtaining this type of material is the following:
To a salt solution is added a portion of gelatine under stirring, in order to yield solid, homogeneous and elastic gelatine. The amount of gelatine to be added is related to the characteristics of the salt solutions to be used in each case. Therefore, the amount of added gelatine will depend on the type of organic salt used, and consequently on its water solubility.
The salt used shall be dissolved in water. Its concentration shall be in the range from 4 to 50% (w/v), this depending on the water solubility of the salt. At this point, the gelatine is to be incorporated in the system until an elastic and solid phase is obtained. The addition of gelatine shall be made gradually, in order to ensure that the mixture remains homogeneous between each of the additions of gelatine. In certain cases, water must be added to the system in order to provide a better homogenization of all the phases, as well as to allow for an improved interaction between the gelatine and the salt.
During this process, parameters such as the temperature, ionic strength and pH shall be monitored. The temperature is a parameter which must be controlled during the whole process. This affects the solubility of gelatine in the salt solution, as well as the cross-linking. The process is faster at a temperature from 60 to 90°C, but it will work from 20 to 90°C. The ionic strength is directly associated with the degree of cross-linking between the gelatine and the salt. Therefore, the ionic strength depends both on the amount of gelatine, and on the type of salt used for manufacturing the material.
In a typical process, the salt/gelatine ratio will vary from 0.5 to 10, and the corresponding ionic strength will vary from 1 M to 60 M. The type of salt can have an influence on the pH of the mixture, and so it is vital to check the pH of the solution prior to manufacturing the material. The material can be produced at any pH value, but the manufacturing process works better if it is within the range from 5 to 9. Another aspect to be considered is the stirring of the mixture. This stirring shall make all the phases to be adequately homogenized. Several types of stirring (orbital, magnetic, mechanical) were assayed. All of them gave very satisfactory results.
The final form of the material is determined by the surface where the gelification occurs. So, one can obtain thin films, compact blocks, or particles in case there is a dispersion of the material formed. The physical properties of the materials, such as colour and opacity, elasticity and conductivity, depend on the type of ionic liquid used and on the final form of the material.
Thus, the first object of the invention is the synthesis of new materials based on gelatine and organic salts, characterized in that the cross-linking of between the gelatine unit and the organic salts (I) 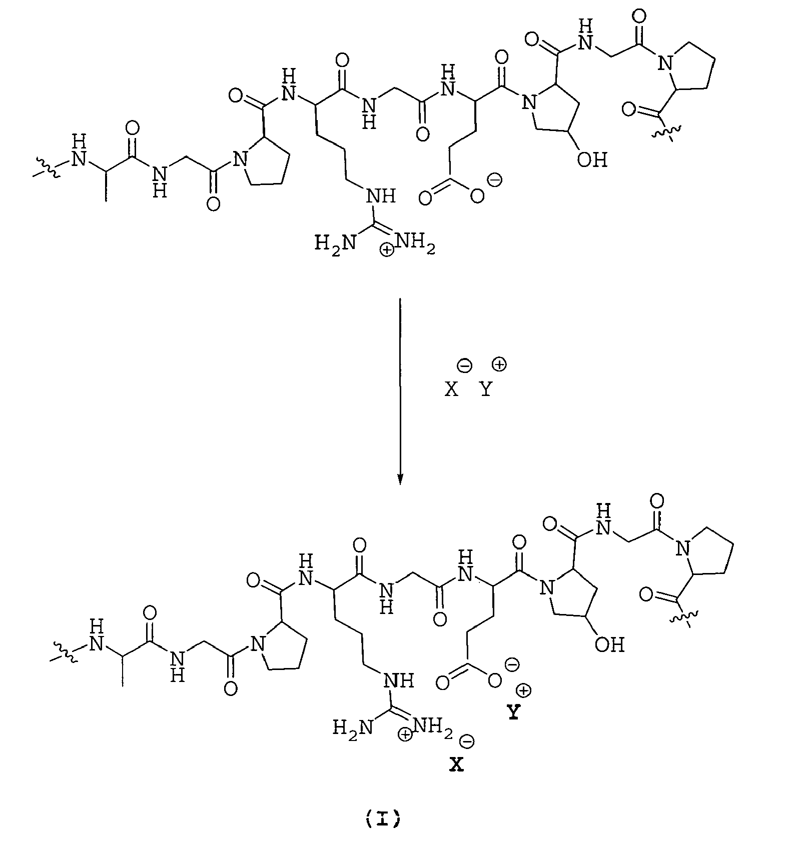
wherein X^{⊖} represents the anion of the salt and Y^{⊕} represents the cation of the salt, is carried out in aqueous solutions with different ionic strengths (0.01-1000 M) and/or different pHs (1-14), depending on the origin of the gelatine used, at a temperature from 20° to 90°C under stirring (magnetic, orbital, mechanical or other).

The gelatine may be of animal, microbial or vegetal origin from the different types A, B, I, II, III, IV.

The organic salts may be in the liquid or solid state and are chiral or achiral.

The organic anion is selected from the following:
a) halides (Cl, Br, I, F), phosphites and phosphates (PF₆, H₂PO₂ H₂PO₄, PO₃), borates (BF₄, BO₂, BO₃), nitrites and nitrates (NO₃, NO₂), sulphates (HSO₄), cyanides and cyanates (CN, SCN, CNO), cyanamides (C₂N₃), dicyanamide (N(CN)₂), azides (N₃), carbonates (HCO₃), bromates (BrO₃), iodates (IO₃, IO₄), chlorites and chlorates (Cl₂O₃, ClO₄, ClO₂, C10) ;
b) metal halides such as ZnCl₃, ZnBr₃, SnCl₅, SnBr₅, FeCl₄, FeBr₄, NiCl₃ , AuBr₄, AuBr₄, AuCl₄, GaCl₄, AlCl₄, Al₂Cl₇, Al3Cl₁₀;
c) compounds which include other elements of the periodic table, preferably from the groups Ib to VIIb, groups IIIa to VIa and group VIII, more preferably including elements such as arsenic (AsF₆, H₂AsO₃ , AsO₂), antimony (SbF₆, SbO₃), chromium (HCrO₄), tellurium (HTeO₆, HteO₃), selenium (HSeO_{3,} SeCN) , niobium (NbO₃), thallium (TaO₃), ruthenium (RuO₄), manganese (MnO₄) and rhenium (ReO₄), bismuth (BiO₃), vanadium (VO₄) and silver (Ag(CN)₂);

### ii) an organic anion selected from the following:

a) carboxylates, thiocarboxylates, carbamates, dithiocarbamates, xanthates, sulphonates, organosulfates, organosulfamates, organophosphates, phosphonates, thiophosphonates and other compounds having the general formula

   R¹-Z-Y⁻ (II)

   wherein Z is CY, SO₂, P(Y)R₂ or As(Y)R_{2;} Y is, independently for each occurrence, O or S;
   and R¹ and R² are equal or different radicals defined as:
   a1) H- or E-, in which E is F, Cl, OH, NH₂ or SH;
   a2) a hydrocarbon radical of 1 to 30 carbon atoms, optionally including double or triple bonds and/or 1 or more saturated, unsaturated or aromatic rings;
   a3) a radical having the same meaning as in a2) but wherein 1 to 15 CH₂ units were substituted by equal or different di-radicals, selected from O, NR³, S, SO, SO₂, CO, SiR₃R₄ or P(O)R₃, the radicals R³ and R⁴ being as defined in paragraphs a1), a2), a3), a4) and a5);
   a4) a radical having the same meaning as in a2) or a3), wherein 1 to 15 CH units were substituted by equal or different tri-radicals selected from N, SiR₅ or PO, R⁵ being a radical as defined in paragraphs a1), a2), a3), a4) and a5);
   a5) a radical having the same meaning as in a2), a3) or a4), wherein one or more H atoms were substituted by F, Cl, Br, I, OH, SH or NH₂;
   being understood that, in case there is a group R² in (II), it can be connected to R¹ by one or more single, double or triple covalent bonds, forming one or more rings, including aromatic rings;
b) imides, thioimides, sulfonimides, N-acyl-sulfonamides, N-acyl-phosphoramides and other compounds of general formula 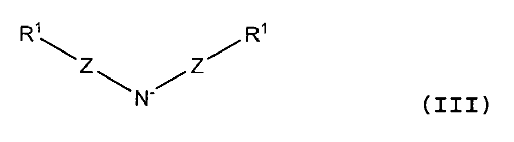 wherein Z is CY, SO₂, P(Y)R₂ or As(Y)R₂; Y is, independently for each occurrence, O or S; and R¹ and R² are, independently for each occurrence, equal or different radicals as defined in paragraphs a1), a2), a3), a4) and a5), being understood that in (III) the groups R¹, and R² if existing, are connected to each other by one or more single, double or triple covalent bonds, forming one or more rings, including aromatic rings;
c) ascorbates, barbiturates, ferrocenecarboxylates, isocyanurates, oxaloacetates, methane-fullerene carboxylates, and mixtures of the compounds mentioned in paragraphs a) and b);

### iii) an organic cation selected from the following:

a1) heterocycles of charge centered in the sulphur, phosphorous, nitrogen, oxygen atoms such as phosphonium, ammonium (including chirals), sulfonium, pyridinium, 8-alkyl-1,8-diazabicyclo[5,4,0]-7-undecenium [alkyl-DBU], 1,2-dialkylimidazolium, 1,3-dialkylimidazolium, 1,2,3-trialkylimidazolium, monoalkyldialkylguanidinium, trialkyldialkylguanidinium, tetraalkyldialkylguanidinium, pentaalkyldialkylguanidinium, hexaalkyldialkylguanidinium;
a2) compounds including other elements of the periodic table, preferably alkaline-earth metals.

The new materials based on gelatine and organic salts are, preferably, in the form of a:
i) solid (fibre, nanofibre, particle, nanoparticle, film, nanofilm, among others)
ii) liquid
iii) colloid (emulsion, foam, gel, aerosol, aerogel, among others)

A second object of the invention is the use of the new materials based on gelatine and organic salts, synthesized as referred to above, selected from one of the following uses:
a) as cell growth medium,
b) in the manufacturing of biosensors and electrodes for bioelectrochemical applications,
c) in the immobilization of chemical and biological catalysts so that chemical reactions are performed,
d) for the impregnation and/or encapsulation of substances having pharmacological properties,
e) as catalysts for chemical transformation reactions in aqueous and non-aqueous media,
f) in the manufacturing and modification of fibres for incorporation of electronic devices into textiles,
g) in electrochemical (galvanic or electrolytic) cells or parts of cells, including fuel cells, so that they can act as conductors or semiconductors,
h) for the manufacture of photovoltaic cells that can act as immobilization matrix for photosynthetic cells, conductive or semi-conductive or electrolytic material,
i) as a new type of membrane employed in the selective transport of different organic molecules (such as alcohols, amines, ethers, esters, carboxylic acids), peptides, amino acids, sugars, anthocyanines, nucleic acids and other compounds of chemical, biochemical or pharmaceutical interest, and
j) as immobilization matrix for cells (of microbial, vegetal and animal origin) in the manufacture of compounds with chemical, biochemical or pharmaceutical interest.

### Example 1

### Cross-linking between gelatine and 1-butyl 3-methyl imidazolium chloride salt (BMIMCl)

To 540 mg of BMIMCl is added a portion of 100 mg of gelatine under magnetic stirring at a temperature in the range from 40 to 90°C, most preferably at 65°C. This mixture shall remain over a period of no less than 60 minutes, most preferably 30 minutes, until it is perfectly homogenous. The mixture is cooled to a temperature from 20° to 40°C. Water is added so that the concentration of salt is from 1 to 250 M, most preferably 155 M. It shall be checked if the pH is at a value from 5 to 9. Gelatine is gradually added in order to keep the salt/gelatine ratio (w/w) in the range from 0.1 to 10, most preferably 1.4. The stirring continues until the mixture is homogenous and then it is allowed to solidify.

### Example 2

### Cross-linking between gelatine and 1-butyl 3 methyl imidazolium dicyanamide salt (BMIMDCA).

To 300 *µ*L of BMIMDCA is added a portion of 30 mg of gelatine under magnetic stirring at a temperature in the range from 40 to 90°C, most preferably 70°C.

This mixture shall remain over a period of no less than 60 minutes, most preferably 30 minutes, until it is perfectly homogenous. The mixture is cooled to a temperature from 20° to 40°C. Water is added so that the concentration of salt is from 1 to 250 M, most preferably 24 M. It shall be checked if the pH is at a value from 5 to 9. Gelatine is gradually added in order to keep the salt/gelatine ratio (w/w) in the range from 0.1 to 10, most preferably 1.4. The stirring continues until the mixture is homogenous and then it is allowed to solidify.

### Example 3

### Cross-linking between gelatine and 1-butyl 3-methyl

### imidazolium saccharin salt (BMIMSaccharin).

To 300 µL of BMIMsaccharin is added a portion of 30 mg of gelatine under magnetic stirring at a temperature in the range from 40 to 90°C, most preferably 70°C.

This mixture shall remain over a period of no less than 60 minutes, most preferably 45 minutes, until it is perfectly homogenous. The mixture is cooled to a temperature from 20° to 40°C. Water is added so that the concentration of salt is from 1 to 250 M, most preferably 14 M. It shall be checked if the pH is at a value from 5 to 9. Gelatine is gradually added in order to keep the salt/gelatine ratio (w/w) is in the range from 0.1 to 10, most preferably 0.8. The stirring continues until the mixture is homogenous and then it is allowed to solidify.

### Example 4

### Cross-linking between gelatine and trioctylmethylammonium dicyanamide salt (ALiqDCA).

To 200 µL of ALiqDCA is added a portion of 20 mg of gelatine under magnetic stirring at a temperature in the range from 40 to 90°C, most preferably 60°C.

This mixture shall remain over a period of no less than 60 minutes, most preferably 45 minutes, until it is perfectly homogenous. The mixture is cooled to a temperature from 10° to 40°C. Water is added so that the concentration of salt is from 0.5 to 800 M, most preferably 150 M. It shall be checked if the pH is at a value from 5 to 9. Gelatine is gradually added in order to keep the salt/gelatine ratio (w/w) in the range from 1 to 50. The stirring continues until the mixture is homogenous and then it is allowed to solidify.

### Example 5

### Cross-linking between gelatine and 1-octyl 3-methyl imidazolium dicyanamide salt (OMIMDCA).

To 200 µL of OMIMDCA is added a portion of 20 mg of gelatine under magnetic stirring at a temperature in the range from 20 to 90°C.

This mixture shall remain over a period of no less than 60 minutes, most preferably 30 minutes, until it is perfectly homogenous. The mixture is cooled to a temperature from 10° to 40°C. Water is added so that the concentration of salt is from 0.5 to 500 M, most preferably 30 M. It shall be checked if the pH is at a value from 5 to 9. Gelatine is gradually added in order to keep the salt/gelatine ratio (w/w) in the range from 1 to 50, most preferably 5. The stirring continues until the mixture is homogenous and then it is allowed to solidify.

### Example 6

### Cross-linking between gelatine and 1-octyl 3-methyl imidazolium chloride salt (OMIMCl).

To 210 µL of OMIMDCA is added a portion of 20 mg of gelatine under magnetic stirring at a temperature in the range from 20 to 90°C.

This mixture shall remain over a period of no less than 60 minutes, most preferably 30 minutes, until it is perfectly homogenous. The mixture is cooled to a temperature from 10° to 40°C. Water is added so that the concentration of salt is from 1 to 250 M, most preferably 6 M. It shall be checked if the pH is at a value from 5 to 9. Gelatine is gradually added in order to keep the salt/gelatine ratio (w/w) in the range from 0.1 to 10. The stirring continues until the mixture is homogenous and then it is allowed to solidify.

### Example 7 Cross-linking between gelatine and sodium dicyanamide salt (NaDCA)

100 mg of NaDCA are dissolved in 190 µL of water under magnetic stirring and at a temperature in the range from 20 to 90°C, preferably 35°C, in order to provide a solution 11.2 M. This mixture shall remain under stirring until it gives a perfectly homogenous solution. The mixture is cooled to a temperature from 10° to 40°C. It shall be checked if the pH is at a value from 5 to 9. Gelatine is gradually added in order to keep the salt/gelatine ratio (w/w) in the range from 0.1 to 100, most preferably 0.9. The stirring continues until the mixture is homogenous. The material shall be dried according to the desired application. Therefore, it can be done by hot air drying, with a temperature from 20 to 90°C, most preferably 35°C, under vacuum, lyophilisation or supercritical extraction.

### Example 8

### Cross-linking between gelatine and sodium saccharin salt (NaSaccharin)

500 mg of NaSaccharin are dissolved in 450 µL of water under magnetic stirring and at a temperature in the range from 20 to 90°C, preferably 35°C, in order to provide a solution 11.2 M. This mixture shall remain under stirring until it gives a perfectly homogenous solution. The mixture is cooled to a temperature from 20° to 40°C. It shall be checked if the pH is at a value from 5 to 9. Gelatine is gradually added in order to keep the salt/gelatine ratio (w/w) in the range from 0.1 to 10, most preferably 1.7. The stirring continues until the mixture is homogenous. The material shall be dried according to the desired application. Therefore, it can be done by hot air drying, with a temperature from 25 to 90°C, most preferably 35°C, under vacuum, lyophilisation or supercritical extraction.

### Example 9

### Cross-linking between gelatine and trioctylmethylammonium chloride salt (AliqCl)

To 200 µL of Aliquat 336^{®} is added a portion of 20 mg of gelatine under magnetic stirring and at a temperature in the range from 20° to 90°C, most preferably 60°C.

This mixture shall remain over a period of no less than 60 minutes, most preferably 30 minutes, until it is perfectly homogenous. The mixture is cooled to a temperature from 10° to 40°C. Water is added so that the concentration of salt is from 0.5 to 800 M, most preferably 150 M. It shall be checked if the pH is at a value from 5 to 9. Gelatine is gradually added in order to keep the salt/gelatine ratio (w/w) in the range from 1 to 50. The stirring continues until the mixture is homogenous and solid.

### Example 10

### Cross-linking between gelatine and a mixture of sodium saccharin (NaSaccharin) and 1-butyl 3-methyl imidazolium dicyanamide salts (BMIMDCA).

1 g of NaSaccharin and 1 mL of BMIMDCA are dissolved in 1 mL of water under magnetic stirring at a temperature from 20° to 90°, preferably 35°C, in order to provide a solution 5.4 M. This mixture shall remain under stirring until it gives a perfectly homogenous solution. The mixture is cooled to a temperature from 10° to 40°C. It shall be checked if the pH is at a value from 5 to 9. Gelatine is gradually added in order to keep the salt/gelatine ratio (w/w) in the range from 0.1 to 10, most preferably 1.7. The stirring continues until the mixture is homogenous. The material shall be dried according to the desired application. Therefore, the drying can take place at room temperature, under vacuum or under supercritical extraction. The final aspect of the material will depend on the drying process.

### References

[1] Ray, S. S.; Bousmina, M. Progress in Materials Science 2005, 50, 962-1079
[2] Y. Tabata e Y. Ikada, Advanced Drug Delivery Reviews, 1998, 31, 287-301
[3] G. A. Digenis, T. B. Gold e V. P. Shah, Journal of Pharmaceutical Sciences, 1994, 83, 915-921; R. Narayani e K. P. Rao, Journal of Biomaterials Science-Polymer Edition, 1995, 7, 39-48.
[4] S. K. Bae, T. H. Sung e J. D. Kim, Journal of Adhesion Science and Technology, 2002, 16, 361-372; H. W. Sung, D. M. Huang, W. H. Chang, L. L. H. Huang, C. C. Tsai e I. L. Liang, Journal of Biomaterials Science-Polymer Edition, 1999, 10, 751-771; H. W. Sung, D. M. Huang, W. H. Chang, R. N. Huang e J. C. Hsu, Journal of Biomedical Materials Research, 1999, 46, 520-530.
[5] Bigi, A., Cojazzi G., Panzavolta, S., Rubini, K. e Roveri, N. Mechanical and thermal properties of gelatin films at different degrees of GTA crosslinking, Biomaterials, 2001, 22, 763-768
[6] A. J. Kuijpers, G. H. M. Engbers, J. Krijgsveld, S. A. J. Zaat, J. Dankert e J. Feijen, Journal of Biomaterials Science-Polymer Edition, 2000, 11, 225-243.
[7] R. Cortesi, C. Nastruzzi e S. S. Davis, Biomaterials, 1998, 19, 1641-1649
[8] M. A. Vandelli, M. Romagnoli, A. Monti, M. Gozzi, P. Guerra, F. Rivasi e F. Forni, Journal of Controlled Release, 2004, 96, 67-84
[9] Y. Z. Zhang, J. Venugopal, Z. M. Huang, C. T. Lim e S. Ramakrishna, Polymer, 2006, 47, 2911-2917
[10] a) R. Sheldon, Chem. Commun. 2001, 2399. b) C. M. Gordon, Applied Catalysis A: General 2001, 222, 101.
[11] M. J. Earle, J. Esperanca, M. A. Gilea, J. N. C. Lopes, L. P. N. Rebelo, J. W. Magee, K. R. Seddon e J. A. Widegren, Nature, 2006, 439, 831-834
[12] X. B. Lu, Q. Zhang, L. Zhang e J. H. Li, Electrochemistry Communications, 2006, 8, 874-878; M. S. P. Lopez, D. Mecerreyes, E. Lopez-Cabarcos e B. Lopez-Ruiz, Biosensors & Bioelectronics, 2006, 21, 2320-2328.
[13] E. D. Bates, R. D. Mayton, I. Ntai, J. H. Davis Jr, J. Am. Chem. Soc. 2002, 124, 926.
[14] F. Kubota e M. Goto, Solvent Extraction Research and Development-Japan, 2006, 13, 23-26.
[15] S. A. A. Rizvi e S. A. Shamsi, Analytical Chemistry, 2006, 78, 7061-7069; D. W. Armstrong, Abstracts of Papers of the American Chemical Society, 2006, 231.
[16] a) L. C. Branco, J. G. Crespo, C. A. M. Afonso, Angew. Chem. Int. Ed. 2002, 41, 2771. b) L. C. Branco, C. A. M. Afonso, Chem. Eur. J. 2002, 8, 3865. c) A. Aggarwal, N. L. Lancaster, A. R. Sethi, T. Welton Green Chem. 2002, 4, 517.
[17] T. Schäfer, C. M. Rodrigues, C. A. M. Afonso, J. G. Crespo, Chem. Commun., 2001, 1622.
[18] R. P. Swatloski, S. K. Spear, J. D. Holbrey, R. D. Rogers, J. Am. Chem. Soc. 2002, 124, 4974.
[19] A. Fernicola, B. Scrosati e H. Ohno, Ionics, 2006, 12, 95-102.
[20] P. Ludley, N. Karodia, Tetrahedron Lett. 2001, 42, 2011.
[21] a) A. P. Abbott, G. Capper, D. L. Davies, H. L. Munro, R. K. Rasheed, V. Tambyrajah, Chem. Comm. 2001, 2010. b) H. Matsumoto, H. Kageyama, Y. Miyazaki, Chem. Comm. 2002, 1726. c) D. Demberelnyamba, B. K. Shin, H. Lee, Chem. Comm. 2002, 1538.
[22] P. Wasserscheid, A. Bösmann, C. Bolm, Chem. Comm. 2002, 200.
[23] H. Matsumoto, T. Matsuda, Y. Miyazaki, Chem. Lett. 2000, 1430.
[24] T. Kitazume, F. Zulfigar, G. Tanaka, Green. Chem. 2000, 2, 133.
[25] L. C. Branco, P. M. P. Góis, N. M. T. Lourenço, V. B. Kurteva e C. A. M. Afonso, Chemical Communications, 2006, 2371-2372; Y. Gao, S. W. Arritt, B. Twamley e J. M. Shreeve, Inorganic Chemistry, 2005, 44, 1704-1712.
[26] J. Kagimoto, K. Fukumoto e H. Ohno, Chemical Communications, 2006, 2254-2256.
[27] a) T. Welton, Chem. Rev. 1999, 99, 2071. b) P. Wasserscheid, W. Keim, Angew. Chem. Int. Ed. 2000, 39, 3772. c) J. Dupont, C. S. Consorti, J. Spencer, J. Braz. Chem. Soc. 2000, 11, 337. d) J. F. Brennecke, E. J. Maginn AIChE Journal 2001, 47, 2384.
[28] a) A. P. Abbott, G. Capper, D. L. Davies, H. L. Munro, R. K. Rasheed, V. Tambyrajah, Chem. Comm. 2001, 2010. b) H. Matsumoto, H. Kageyama, Y. Miyazaki, Chem. Comm. 2002, 1726. c) P. Ludley, N. Karodia, Tetrahedron Lett. 2001, 42, 2011. d) S. I. Lall, D. Mancheno, S. Castro, V. Behaj, J. L. I. Cohen, R. Engel, Chem. Comm. 2000, 2413. e) A. S. Larsen, J. D. Holbrey, F. S. Tham, C. A. Reed, J. Am. Chem. Soc. 2000, 122, 7264.

## Claims

1. The synthesis of new materials based on gelatine and organic salts, **characterized in that** the cross-linking of between the gelatine unit and the organic salts (I) 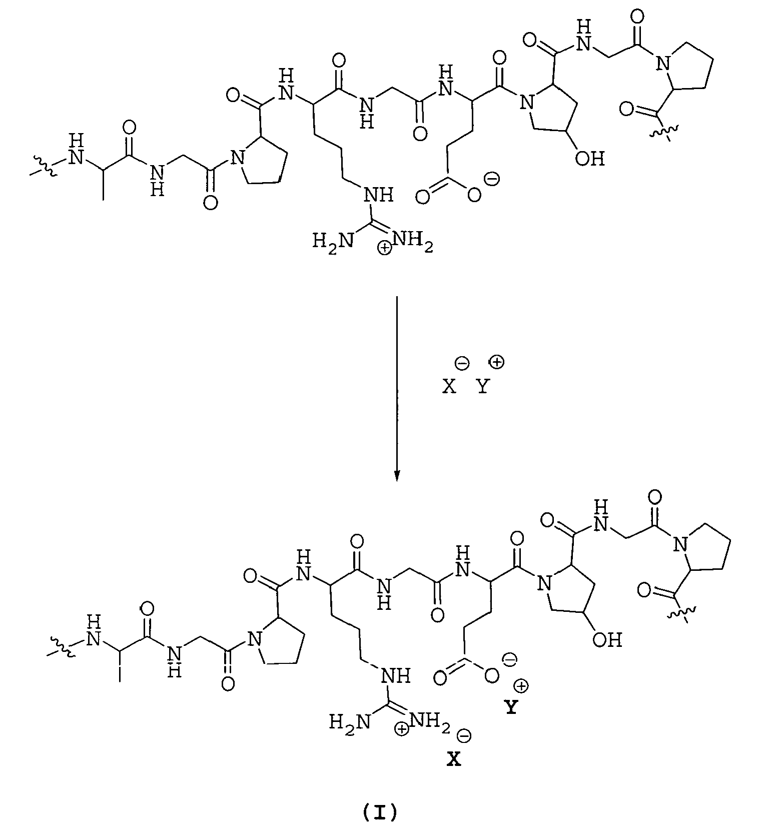 wherein X^{⊖} represents the anion of the salt and Y^{⊕} represents the cation of the salt, is carried out in aqueous solutions with different ionic strengths (0.01-1000 M) and/or different pHs (1-14), depending on the origin of the gelatine used, at a temperature from 20° to 90°C under stirring (magnetic, orbital, mechanical or other).

2. The synthesis of new materials based on gelatine and organic salts according to claim 1, **characterized in that** the gelatine is of animal, microbial or vegetal origin from the different types A, B, I, II, III, IV.

3. The synthesis of new materials based on gelatine and organic salts according to claims 1 and 2, **characterized in that** the organic salts are in the liquid or solid state.

4. The synthesis of new materials based on gelatine and organic salts according to claims 1 to 3, **characterized in that** the organic salts are chiral or achiral.

5. The synthesis of new materials based on gelatine and organic salts according to claims 1 to 4, **characterized in that** an anion X⁻ and a cation Y⁺ are present, which can be:
i) an organic anion selected from the following:
a) halides (Cl, Br, I, F), phosphites and phosphates (PF₆, H₂PO₂ H₂PO₄, PO₃), borates (BF₄, BO₂, BO₃), nitrites and nitrates (NO₃, NO₂), sulphates (HSO₄), cyanides and cyanates (CN, SCN, CNO), cyanamides (C₂N₃), dicyanamide (N(CN)₂), azides (N₃), carbonates (HCO₃), bromates (BrO₃), iodates (IO₃, IO₄), chlorites and chlorates (Cl₂O₃, ClO₄, ClO₂, ClO);
b) metal halides such as ZnCl₃, ZnBr₃, SnCl₅, SnBr₅, FeCl₄, FeBr₄, NiCl₃, AuBr₄, AuBr₄, AuCl₄, GaCl₄, AlCl₄, Al₂Cl₇, Al3Cl₁₀;
c) compounds which include other elements of the periodic table, preferably from the groups Ib to VIIb, groups IIIa to VIa and group VIII, more preferably including elements such as arsenic (AsF₆, H₂AsO₃, AsO₂) antimony (SbF₆, SbO₃), chromium (HCrO₄), tellurium (HTeO₆, HteO₃), selenium (HSeO₃, SeCN), niobium (NbO₃), thallium (TaO₃), ruthenium (RuO₄), manganese (MnO₄) and rhenium (ReO₄), bismuth (BiO₃), vanadium (VO₄) and silver (Ag(CN)₂);
ii) an organic anion selected from the following :
a) carboxylates, thiocarboxylates, carbamates, dithiocarbamates, xanthates, sulphonates, organosulfates, organosulfamates, organophosphates, phosphonates, thiophosphonates and other compounds having the general formula
R¹-Z-Y- (II)
wherein Z is CY, SO₂, P (Y) R₂ or As (Y) R₂; Y is, independently for each occurrence, O or S; and R¹ and R² are equal or different radicals defined as:
a1) H- or E-, in which E is F, Cl, OH, NH₂ or SH;
a2) a hydrocarbon radical of 1 to 30 carbon atoms, optionally including double or triple bonds and/or 1 or more saturated, unsaturated or aromatic rings;
a3) a radical having the same meaning as in a2) but wherein 1 to 15 CH₂ units were substituted by equal or different di-radicals, selected from O, NR³, S, SO, SO₂, CO, SiR₃R₄ or P(O)R₃, the radicals R³ and R⁴ being as defined in paragraphs a1), a2), a3), a4) and a5);
a4) a radical having the same meaning as in a2) or a3), wherein 1 to 15 CH units were substituted by equal or different tri-radicals selected from N, SiR₅ or PO, R⁵ being a radical as defined in paragraphs a1), a2), a3), a4) and a5);
a5) a radical having the same meaning as in a2), a3) or a4), wherein one or more H atoms were substituted by F, Cl, Br, I, OH, SH or NH₂;
being understood that, in case there is a group R² in (II), it can be connected to R¹ by one or more single, double or triple covalent bonds, forming one or more rings, including aromatic rings;
b) imides, thioimides, sulfonimides, N-acyl-sulfonamides, N-acyl-phosphoramides and other compounds of general formula 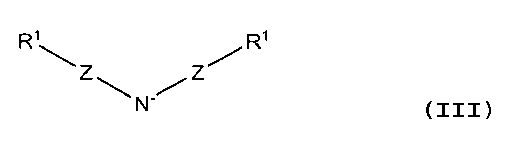 wherein Z is CY, SO₂, P(Y)R₂ or As (Y)R₂; Y is, independently for each occurrence, O or S; and R¹ and R² are, independently for each occurrence, equal or different radicals as defined in paragraphs a1), a2), a3), a4) and a5), being understood that in (III) the groups R¹, and R² if existing, are connected to each other by one or more single, double or triple covalent bonds, forming one or more rings, including aromatic rings;
c) ascorbates, barbiturates, ferrocenecarboxylates, isocyanurates, oxaloacetates, methane-fullerene carboxylates, and mixtures of the compounds mentioned in paragraphs a) and b);
iii) an organic cation selected from the following:
a1) heterocycles of charge centered in the sulphur, phosphorous, nitrogen, oxygen atoms such as phosphonium, ammonium (including chirals), sulfonium, pyridinium, 8-alkyl-1,8-diazabicyclo[5,4,0]-7-undecenium [alkyl-DBU], 1,2-dialkylimidazolium, 1,3-dialkylimidazolium, 1,2,3-trialkylimidazolium, monoalkyldialkylguanidinium, trialkyldialkylguanidinium, tetraalkyldialkylguanidinium, pentaalkyldialkylguanidinium, hexaalkyldialkylguanidinium;
a2) compounds including other elements of the periodic table, preferably alkaline-earth metals.

6. The synthesis of new materials based on gelatine and organic salts according to claims 1 to 5, **characterized in that** the materials are in the form of a:
i) solid (fibre, nanofibre, particle, nanoparticle, film, nanofilm, among others)
ii) liquid
iii) colloid (emulsion, foam, gel, aerosol, aerogel, among others)

7. Use of the new materials based on gelatine and organic salts, which are synthesized according to claims 1 to 6, **characterized in that** the materials are used as cell growth medium.

8. Use of the new materials based on gelatine and organic salts, which are synthesized according to claims 1 to 6, **characterized in that** the materials are used in the manufacturing of biosensors and electrodes for bioelectrochemical applications.

9. Use of the new materials based on gelatine and organic salts, which are synthesized according to claims 1 to 6, **characterized in that** the materials are used in the immobilization of chemical and biological catalysts so that chemical reactions are performed.

10. Use of the new materials based on gelatine and organic salts, which are synthesized according to claims 1 to 6, **characterized in that** the materials are used for the impregnation and/or encapsulation of substances having pharmacological properties.

11. Use of the new materials based on gelatine and organic salts, which are synthesized according to claims 1 to 6, **characterized in that** the materials are used as catalysts for chemical transformation reactions in aqueous and non-aqueous media.

12. Use of the new materials based on gelatine and organic salts, which are synthesized according to claims 1 to 6, **characterized in that** the materials are used in the manufacturing and modification of fibres for incorporation of electronic devices into textiles.

13. Use of the new materials based on gelatine and organic salts, which are synthesized according to claims 1 to 6, **characterized in that** the materials are used in electrochemical (galvanic or electrolytic) cells or parts of cells, including fuel cells, so that they can act as conductors or semiconductors.

14. Use of the new materials based on gelatine and organic salts, which are synthesized according to claims 1 to 6, **characterized in that** the materials are used for the manufacture of photovoltaic cells that can act as immobilization matrix for photosynthetic cells, conductive or semi-conductive or electrolytic material.

15. Use of the new materials based on gelatine and organic salts, which are synthesized according to claims 1 to 6, **characterized in that** the materials are used as a new type of membrane employed in the selective transport of different organic molecules (such as alcohols, amines, ethers, esters, carboxylic acids), peptides, amino acids, sugars, anthocyanines, nucleic acids and other compounds of chemical, biochemical or pharmaceutical interest.

16. Use of the new materials based on gelatine and organic salts, which are synthesized according to claims 1 to 6, **characterized in that** the materials are used as immobilization matrix for cells (of microbial, vegetal and animal origin) in the manufacture of compounds with chemical, biochemical or pharmaceutical interest.
